# EUROPEAN PATENT APPLICATION

(11) **EP 1 922 976 A1**
(43) Date of publication of application: **21.05.2008**
(21) Application number: 06783228.7
(22) Date of filing: 08.09.2006
(51) Int. Cl.: A61B 1/00, A61B 1/04, A61B 5/07, G06F 13/38

(54) **RECEIVER APPARATUS, MONITOR APPARATUS, AND INTRA-SUBJECT INFORMATION ACQUIRING SYSTEM USING THEM**

(30) Priority: 09.09.2005 JP 2005263113
(71) Applicant: OLYMPUS MEDICAL SYSTEMS CORP., Shibuya-ku Tokyo 151-0072 (JP)
(72) Inventor: NAGASE, Ayako c/o Olympus Medical Systems Corp.,, Tokyo 151-0072 (JP)
(74) Representative: von Hellfeld, Axel
(86) International application number: PCT/JP2006/317868
(87) International publication number: WO 2007/029818

(57) **Abstract**

An object of the present invention is to allow for correctly confirming whether image data is stored or not, and to allow for downsizing of an apparatus scale. An intra-subject information acquiring system according to the present invention includes a receiving apparatus 3 that acquires image data from a capsule endoscope 2, and a monitor apparatus 7 that displays image data acquired by the receiving apparatus 3. The receiving apparatus 3 includes an image determining unit 40 that determines validity/invalidity of the acquired image data, a data generator 41 that generates data for output in which the result of determination is added to an image signal, and a differential transmission I/F 44 that transmits the data for output to the monitor apparatus 7. The monitor apparatus 7 includes a determination result detector 75 that detects the determination result included in an image signal, which is the data for output from the receiving apparatus 3, and a display unit 76 that displays image data based on the image signal and information corresponding to the determination result.

## Description

### TECHNICAL FIELD

The present invention relates to a receiving apparatus that receives image data acquired through image capturing by a capsule endoscope introduced inside a body of a subject via a predetermined electric wave, a monitor apparatus that performs monitoring of the image data received by the receiving apparatus, and an intra-subject information acquiring system employing the above apparatuses.

### BACKGROUND ART

In recent years, a capsule endoscope, which is a swallowable endoscope equipped with an imaging function and a radio communication function appears in a field of endoscope, and an intra-subject information acquiring system which acquires image data acquired through image capturing by the capsule endoscope inside a subject is being developed. In the intra-subject information acquiring system, the capsule endoscope travels inside internal organs such as a stomach and a small intestine in the subject following peristaltic movements and works to capture images inside the subject at predetermined intervals, for example, at 0.5-second intervals, after being swallowed from a mouth of the subject for an observation (examination) until naturally discharged from the subject.

The image data acquired through image capturing by the capsule endoscope during the travel inside the subject is sequentially transmitted to an outside by radio communication, and received by a receiving apparatus via receiving antennas arranged outside the subject in a dispersed manner. The receiving apparatus demodulates radio signals received by the receiving antennas into image signals, and generates image data by performing predetermined image processing on the resulting image signals. Thereafter, the receiving apparatus sequentially stores the image data generated in a memory. Since the subject carries the receiving apparatus having a radio communication function and a memory function, the subject can move freely after swallowing the capsule endoscope until naturally discharging the same. Later, a doctor or a nurse can make diagnosis of the subject by displaying the image inside the subject on a screen of a display device based on the image data stored in the receiving apparatus (see Patent Document 1, for example).

Some receiving apparatus of the intra-subject information acquiring system detect horizontal synchronization signals included in the obtained image signals for each frame, determines that the generated image data is valid image data with little noises when the number of detected horizontal synchronization signals is equal to or larger than a predetermined threshold, and determines that the generated image data is invalid image data with many noises when the number of detected horizontal synchronization signals is less than the predetermined threshold. The receiving apparatus sequentially stores the valid image data in a memory without storing the invalid image data.

Further, a monitor apparatus which displays the image data acquired through image capturing by the capsule endoscope on a screen is connected to the receiving apparatus of the intra-subject information acquiring system via a cable. The monitor apparatus receives respective color signals (R, G, B) and synchronization signals of the valid image data stored in the receiving apparatus from the receiving apparatus and sequentially displays the valid image data on a screen based on the received respective color signals and the synchronization signals. The doctor, the nurse, or the like can monitor the valid image data stored in the receiving apparatus on real-time by confirming the images displayed on the monitor apparatus.

Patent Document 1: Japanese Patent Application Laid-Open No. 2003-19111

### DISCLOSURE OF INVENTION

### PROBLEM TO BE SOLVED BY THE INVENTION

The receiving apparatus of the intra-subject information acquiring system is carried by the subject while the capsule endoscope captures images inside the body. Therefore, further downsizing of the apparatus scale is desired.

The receiving apparatus, however, is required to incorporate necessary circuits, for example, a crystal oscillator, and an operational amplifier, for generating the respective color signals and the synchronization signals of the image data transmitted to the monitor apparatus. Therefore, there is a certain limitation to the downsizing of the circuit scale in the receiving apparatus. Further, it is necessary to connect the receiving apparatus and the monitor apparatus at least with four cables for the transmission of the respective color signals of R, G, and B, and the synchronization signals from the receiving apparatus to the monitor apparatus. Thus, the downsizing of the apparatus scale of the receiving apparatus is difficult to realize.

If the image signals described above are transmitted in serial form from the receiving apparatus to the monitor apparatus for the reduction of the number of cables and the circuit scale of the receiving apparatus, the monitor apparatus sequentially displays the image data received from the receiving apparatus on the screen disregarding whether the received image data is stored in the receiving apparatus or not. If the doctor, the nurse, or the like wants to check whether the image data displayed on the screen is stored in the receiving apparatus or not, the monitor apparatus is required to determine whether the image data is valid or invalid based on the number of detected horizontal synchronization signals of the image signals similarly to the receiving apparatus as described above and to display information on the screen indicating whether the displayed image data is stored in the receiving apparatus or not based on the result of determination.

However, the result of determination on the image data by the monitor apparatus can be different from the result of determination by the receiving apparatus, due to sensitivity of the receiving apparatus with respect to the radio signals and tolerance of each crystal oscillator provided in the receiving apparatus and the monitor apparatus. Therefore, the monitor apparatus sometimes cannot correctly display on the screen information indicating whether the image data is stored in the receiving apparatus or not.

In view of the foregoing, an object of the present invention is to provide a receiving apparatus, a monitor apparatus, and an intra-subject information acquiring system employing the above apparatuses, according to which, the image data can be correctly checked whether it is stored or not, and the apparatus scale can be downsized.

### MEANS FOR SOLVING PROBLEM

To solve the problems as described above, and to achieve the objects, a receiving apparatus according to the present invention includes a receiving unit that receives image data; a determining unit that is connected to a subsequent stage of the receiving unit and determines whether the image data acquired is a target of record or not; a data generator that generates data for output to which information on necessity of recording is included, the information being a result of determination by the determining unit; and an interface that outputs the data for output.

Further, in the receiving apparatus according to the present invention as set forth above, the data generator generates the data for output by adding the information on necessity of recording to the image data.

Still further, in the receiving apparatus according to the present invention as set forth above, the interface is a serial interface.

Still further, in the receiving apparatus according to the present invention as set forth above, the serial interface is a differential transmission interface.

Still further, the receiving apparatus according to the present invention as set forth above, includes a binarizing unit that binarizes the image data, and the data generator adds the information on necessity of recording to the image data binarized by the binarizing unit.

Still further, in the receiving apparatus according to the present invention as set forth above, the determining unit detects a horizontal synchronization signal included in an image signal which includes the image data, and determines that the image data is the target of record when a number of the horizontal synchronization signals detected is equal to or larger than a predetermined threshold.

Still further, a monitor apparatus according to the present invention includes an interface that receives radio signals including image data acquired through image capturing by a capsule endoscope introduced into a subject, acquires the image data based on the radio signals, determines whether the image data acquired is valid image data or not, and receives image signals which includes the image data acquired by a receiving apparatus and a result of determination on the image data, the interface being connected to the receiving apparatus which stores the valid image data therein; a detector that detects the result of determination included in the image signal; a display unit that displays the image data and information corresponding to the result of determination, the image data and the information being included in the image signal; and a control unit that controls to display the image data and the information corresponding to the result of determination based on the result of determination detected by the detector, the image data and the information being included in the image signal.

Still further, in the monitor apparatus according to the present invention as set forth above, the interface is a serial interface.

Still further, in the monitor apparatus according to the present invention as set forth above, the serial interface is a differential transmission interface.

Still further, in the monitor apparatus according to the present invention as set forth above, the information corresponding to the result of determination is information indicating whether the image data is stored in the receiving apparatus or not.

Still further, an intra-subject information acquiring system includes a receiving apparatus that receives radio signals including image data acquired through image capturing by a capsule endoscope introduced into a subject, demodulates the radio signals received into image signals, and stores valid image data among the image data acquired based on the image signals; and a monitor apparatus that is connected to the receiving apparatus via an interface, and displays the image data acquired by the receiving apparatus, wherein the receiving apparatus determines whether the image data acquired is a target of record or not, generates data for output including a result of determination and the image data, and transmits the acquired data for output to the monitor apparatus via the interface, and the monitor apparatus receives the data for output via the interface, detects the result of determination included in the data for output received, and displays information corresponding to the result of determination detected together with the image data.

Still further, in the intra-subject information acquiring system according to the present invention as set forth above, the interface is a serial interface.

Still further, in the intra-subject information acquiring system according to the present invention as set forth above, the serial interface is a differential transmission interface.

Still further, in the intra-subject information acquiring system according to the present invention as set forth above, the information corresponding to the result of determination is information indicating whether the image data is stored in the receiving apparatus or not.

### EFFECT OF THE INVENTION

According to the present invention, the image signals on which the determination result on validity/invalidity of the image data is superposed can be transmitted in serial form, the image data can be correctly checked whether it is stored or not, and the downsizing in the apparatus scale of the receiving apparatus, the monitor apparatus, and the intra-subject information acquiring system employing the above apparatuses can be realized.

### BRIEF DESCRIPTION OF DRAWINGS

FIG. 1 is a schematic diagram of an exemplary structure of an intra-subject information acquiring system according to an embodiment of the present invention;
FIG. 2 is a schematic block diagram of an exemplary structure of each of a receiving apparatus and a monitor apparatus according to the embodiment of the present invention;
FIG. 3 is a schematic diagram describing an operation of generating an additional binarization signal in which a determination result of image data is added to a binarization signal;
FIG. 4 is a schematic diagram describing an operation of detecting the determination result included in the additional binarization signal of the image data; and
FIG. 5 is a schematic diagram of a specific example of contents of display by a display unit.

### EXPLANATIONS OF LETTERS OR NUMERALS

- 1: Subject
- 2: Capsule endoscope
- 3: Receiving apparatus
- 4: Display device
- 5: Portable recording medium
- 6a to 6h: Receiving antenna
- 7: Monitor apparatus
- 8: Cable
- 31: Antenna switching unit
- 32: Signal strength detector
- 33: AD converter
- 34: Antenna selector
- 35: Demodulator
- 36: Synchronization detector
- 37: Binarizing unit
- 38,: 43 AND gate
- 39: Image processing unit
- 40: Image determining unit
- 41: Data generator
- 42: Connection detector
- 44: Differential transmission I/F
- 45: Display unit
- 46: Storage unit
- 47: Control unit
- 48: Power supply unit
- 71: Differential transmission I/F
- 72: Connection detector
- 73: Switching circuit
- 74: Image processing unit
- 75: Determination result detector
- 76: Display unit
- 77: Control unit
- 78: Power supply unit
- 80: Receiving antenna
- 81: Demodulator
- 82: Synchronization detector
- 83: Binarizing unit
- 84: AND gate
- 101: Image data
- 102: Storage information
- 103: Connection state information
- 104: Remaining-battery-amount information
- B1, B2: Blanking portion
- D1, D2: Frame data
- R1, R2: Determination result data
- S1: Binarization signal
- S2: Determination result signal
- S3: Additional binarization signal
- S4: Determination result detection signal

### BEST MODE(S) FOR CARRYING OUT THE INVENTION

Exemplary embodiments of a receiving apparatus, a monitoring apparatus, and an intra-subject information acquiring system including the receiving apparatus and the monitor apparatus according to the present invention will be described in detail below with reference to the accompanying drawings. It should be noted that the present invention is not limited to the embodiments.

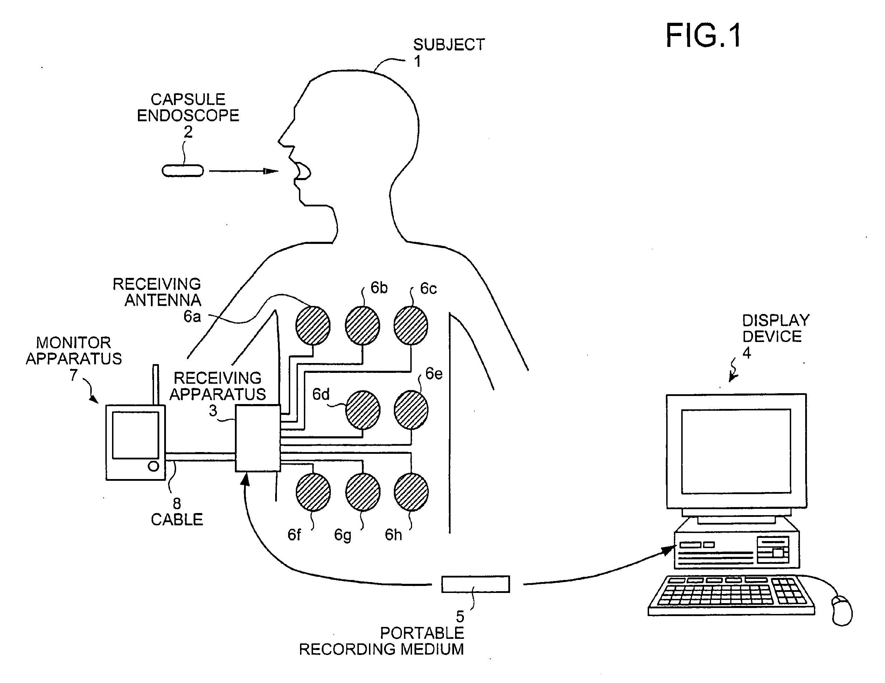

FIG. 1 is a schematic diagram of an exemplary structure of the intra-subject information acquiring system according to the embodiment of the present invention. As shown in FIG. 1, the intra-subject information acquiring system includes a capsule endoscope 2 that travels along a passage in a subject 1 and captures images inside the subject 1, a receiving apparatus 3 that receives image data acquired through image capturing by the capsule endoscope 2, a display device 4 that displays images inside the subject 1 based on the image data acquired through image capturing by the capsule endoscope 2, and a portable recording medium 5 that serves for data delivery between the receiving apparatus 3 and the display device 4.

The capsule endoscope 2 has an imaging function of being capable of capturing images inside the subject and a radio communication function of transmitting acquired image data to the outside. Specifically, the capsule endoscope 2, by being swallowed by the subject 1, passes through esophagus of the subject 1 and travels inside the body cavities along the peristaltic movement of the digestive tracts. At the same time, the capsule endoscope 2 sequentially captures images inside the subject 1 and sequentially transmits the acquired image data and the like inside the subject 1 to the receiving apparatus 3 outside the subject 1.

The receiving apparatus 3 serves to accumulate the image data acquired through image capturing by the capsule endoscope 2. Specifically, the receiving apparatus 3 is connected to receiving antennas 6a to 6h, receives radio signals from the capsule endoscope 2 through one of the receiving antennas 6a to 6h, and acquires the image data included in the received radio signals, in other words, the image data acquired through image capturing by the capsule endoscope 2. Here, since the plural receiving antennas are arranged on the subject 1, the receiving apparatus 3 can acquire the image data of the capsule endoscope 2 via a receiving antenna which is at an appropriate position for the reception of the radio signals depending on the position of the capsule endoscope 2 in the subject 1. Further, the receiving apparatus 3 has an image determination function of determining whether the acquired image data is valid or invalid, and sequentially stores an image which is determined to be valid. Specifically, the receiving apparatus 3 sequentially stores acquired valid image data in the portable recording medium 5 which is detachably attached to the receiving apparatus 3.

The receiving antennas 6a to 6h are, for example, realized with loop antennas, and are arranged at predetermined positions (for example, at positions corresponding to the passage of the capsule endoscope 2) on a body surface of the subject 1 as shown in FIG. 1. The receiving antennas 6a to 6h, arranged as described above, receive the radio signals transmitted from the capsule endoscope 2. It is sufficient if one or more receiving antennas, desirably plural receiving antennas are arranged for the subject 1, and the number of receiving antennas is not particularly limited to eight.

The display device 4 serves to display images or the like of the subject 1 as acquired through image capturing by the capsule endoscope 2, and has a structure like a workstation or the like that displays images based on the image data or the like obtained via the portable recording medium 5, e.g., images of organs or the like inside the subject 1. The display device 4 may display images as in a CRT display or a liquid crystal display, and alternatively, the display device 4 may output the images to other media as in a printer. Further, the display device 4 has a processing function to help doctors and nurses make diagnosis based on the images of the organs or the like inside the subject as obtained by the capsule endoscope 2.

The portable recording medium 5 is, for example, a Compact Flash (registered trademark) or the like that is a recording medium and is portable. The portable recording medium 5 can be attached to and detached from the receiving apparatus 3 and the display device 4, and is configured so as to allow for output and recording of the data when attached to the receiving apparatus 3 and the display device 4. Specifically, the portable recording medium 5 sequentially stores the image data or the like acquired by the capsule endoscope 2 and received by the receiving apparatus 3 when attached to the receiving apparatus 3. After the capsule endoscope 2 is discharged from the subject 1, the portable recording medium 5 is removed from the receiving apparatus 3 and attached to the display device 4, so that the image data or the like is taken into the display device 4. Since the data delivery between the receiving apparatus 3 and the display device 4 is performed with the portable recording medium 5, the subject 1 can move freely by carrying the receiving apparatus 3, even while the capsule endoscope 2 travels inside the subject 1, dissimilar to the system where the receiving apparatus 3 and the display device 4 are connected by a cable.

The monitor apparatus 7 serves to display the image data acquired through image capturing by the capsule endoscope 2 on the screen on real time. Specifically, the monitor apparatus 7 sequentially displays the image data acquired by the receiving apparatus 3, for example, the image data acquired through image capturing by the capsule endoscope 2 inside the subject 1, when connected to the receiving apparatus 3 via a cable 8. The monitor apparatus 7 receives the image signals from the receiving apparatus 3 via the cable 8 and displays the image data on the screen based on the received image signals. Further, the monitor apparatus 7 displays information indicating whether the image data displayed on the screen is stored in the receiving apparatus 3 or not together with the image data.

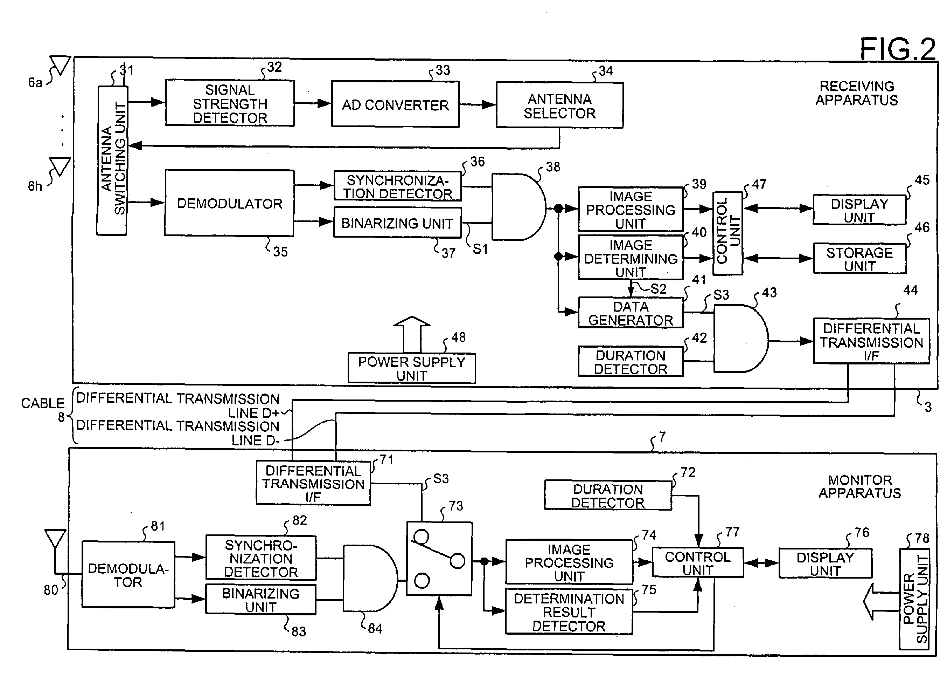

The receiving apparatus 3 and the monitor apparatus 7 connected by the cable 8 will be described in detail. FIG. 2 is a schematic block diagram of an exemplary structure of each of the receiving apparatus 3 and the monitor apparatus 7 according to the embodiment of the present invention. In the following, the receiving apparatus 3 will be described first, followed by the description of the monitor apparatus 7.

As shown in FIG. 2, the receiving apparatus 3 includes the receiving antennas 6a to 6h described earlier, an antenna switching unit 31 that switches to one of the receiving antennas 6a to 6h as a receiving antenna to receive the radio signals from the capsule endoscope 2, a signal strength detector 32 that detects the strength of the radio signal received via the antenna switching unit 31, an AD converter 33 that converts analog signals supplied from the signal strength detector 32 to digital signals, and an antenna selector 34 that selects a receiving antenna appropriate for the reception of the radio signals from the receiving antennas 6a to 6h that can be switched over by the antenna switching unit 31. Further, the receiving apparatus 3 includes a demodulator 35 that demodulates the radio signals received via the antenna switching unit 31 to image signals, a synchronization detector 36 that detects vertical synchronization signals included in the image signal demodulated by the demodulator 35, a binarizing unit 37 that binarizes (in other words, digitizes) the image signals demodulated by the demodulator 35, and an AND gate 38 that controls an output of the image signals binarized by the binarizing unit 37. Further, the receiving apparatus 3 includes an image processing unit 39 that generates image data based on the image signals supplied via the AND gate 38, an image determining unit 40 that determines whether the image data is valid/invalid based on the image signals supplied through the AND gate 38, a data generator 41 that generates data for output, which is an image signal including the image signal supplied via the AND gate 38 and the result of determination by the image determining unit 40, a connection detector 42 that detects a connection between the receiving apparatus 3 and the monitor apparatus 7, an AND gate 43 that controls an output of the image signal which includes the result of determination on the image data as a result of processing in the data generator 41, and a differential transmission interface (I/F) 44 that transmits the image signal including the determination result on the image data to the monitor apparatus 7 according to a differential transmission method in a serial form. Further, the receiving apparatus 3 includes a display unit 45 that has a display function to display various types of information and an information input function implemented with a touch panel or the like, a storage unit 46 that stores various types of information such as image data, a control unit 47 that' controls driving of each element in the receiving apparatus 3, and a power supply unit 48 that supplies driving power to each element in the receiving apparatus 3.

The antenna switching unit 31 serves to switch to one of the plural receiving antennas 6a to 6h as the receiving antenna for receiving the radio signals from the capsule endoscope 2. Specifically, the antenna switching unit 31 is connected to the plural receiving antennas 6a to 6h via a cable, switches to the receiving antenna selected by the antenna selector 34 among the receiving antennas 6a to 6h, and receives the radio signals from the capsule endoscope 2 via the switched receiving antenna. The antenna switching unit 31 supplies the radio signals received via one of the receiving antennas 6a to 6h to the signal strength detector 32 and the demodulator 35.

The signal strength detector 32 serves to detect the strength of the radio signal received via the antenna switching unit 31. Specifically, the signal strength detector 32 detects the strength of the radio signal sent from the capsule endoscope 2 and received via the antenna switching unit 31, and supplies an analog signal, for example, RSSI (Received Signal Strength Indicator) that indicates the detected strength of the received signal to the AD converter 33. The AD converter 33 converts the analog signal of RSSI supplied from the signal strength detector 32 into a digital signal, and supplies the resulting digital RSSI signal to the antenna selector 34.

The antenna selector 34 works to select most appropriate receiving antenna for the reception of the radio signals from the capsule endoscope 2 from the plural receiving antennas 6a to 6h. Specifically, the antenna selector 34 selects a receiving antenna which receives the radio signal with a highest strength from the capsule endoscope 2 from the plural receiving antennas 6a to 6h based on the digital RSSI signal supplied from the AD converter 33, and controls the switching operation of the antenna switching unit 31 so as to switch to the selected receiving antenna.

The demodulator 35 serves to demodulate the radio signals sent from the capsule endoscope 2 and received via the antenna switching unit 31 into image signals. Specifically, the demodulator 35 performs processing such as demodulation on the radio signals sent from the capsule endoscope 2 and received via the antenna switching unit 31, to demodulate the radio signals into the image signals, which are base band signals. The image signal is a serial signal which includes at least image data acquired through image capturing by the capsule endoscope 2, a vertical synchronization signal of each frame, a horizontal synchronization signal of each line in each frame, and parameters such as a white balance coefficient of the image data. The demodulator 35 supplies the resulting image signal to the synchronization detector 36 and the binarizing unit 37.

The synchronization detector 36 serves to detect the vertical synchronization signal included in the image signal demodulated in the demodulator 35. Specifically, the synchronization detector 36 detects the vertical synchronization signal included in the image signal supplied from the demodulator 35 for each frame, and supplies a high-level signal to the AND gate 38 every time the vertical synchronization signal is detected.

The binarizing unit 37 serves to binarize the image signal demodulated by the demodulator 35. Specifically, the binarizing unit 37 performs comparison processing to compare a voltage value of the image signal and a predetermined reference voltage, for example, with respect to the image signal supplied from the demodulator 35, and binarizes the image signal based on the result of comparison processing. The binarizing unit 37 supplies a binarization signal S1, which is a binarized image signal, to the AND gate 38.

The AND gate 38 serves to control the output of the binarization signal S1 to the image processing unit 39, the image determining unit 40, and the data generator 41 in the subsequent stage. Specifically, the AND gate 38 supplies the binarization signal S1 supplied from the binarizing unit 37 when the aforementioned high-level signal is supplied from the synchronization detector 36. In other words, the AND gate 38 supplies the binarization signal S1 to the image processing unit 39, the image determining unit 40, and the data generator 41 at the timing of detection of the vertical synchronization signal of the image signal by the synchronization detector 36.

The image processing unit 39 serves to generate image data based on the binarization signal S1. Specifically, the image processing unit 39 receives the binarization signal S1 via the AND gate 38, and generates the image data on frame basis by performing predetermined image processing and the like on the received binarization signal S1. The generated image data is the image data acquired through image capturing by the capsule endoscope 2 inside the subject 1, for example. The image processing unit 39 outputs the image data thus generated to the control unit 47. Here, the timing of output of the binarization signal S1 is controlled by the AND gate 38, as described above. Hence, the image processing unit 39 can readily detect the horizontal synchronization signal and the vertical synchronization signal of the binarization signal S1 on performing the predetermined image processing on the binarization signal S1.

The image determining unit 40 serves to determine whether the image data generated by the image processing unit 39 is valid or invalid. Specifically, the image determining unit 40 receives the binarization signal S1 via the AND gate 38, detects the horizontal synchronization signals included in the received binarization signal S1 (i.e., the binarized image signal) for each frame, and determine whether the image data is valid/invalid based on the number of detected horizontal synchronization signals of each frame. The image determining unit 40 determines that the image data generated based on the image signal is valid when the number of horizontal synchronization signals detected from the image signal of one frame is equal to or larger than a predetermined threshold, whereas the image determining unit 40 determines that the image data generated based on the image signal is invalid when the number of the detected horizontal synchronization signals is less than the predetermined threshold. Here, the output timing of the binarization signal S1 is controlled by the AND gate 38 as described above. Therefore, the image determining unit 40 can readily detect the horizontal synchronization signals of the binarization signal S1 on determining the validity/invalidity of the image data based on the binarization signal S1. Thereafter, the image determining unit 40 supplies the result of determination of the image data to the control unit 47, and supplies a binarized determination result signal S2 corresponding to the result of determination to the data generator 41. For example, the determination result signal S2 attains a high level indicating the determination result of "validity" when the image data is determined to be valid, whereas the determination result signal S2 attains a low level indicating the determination result of "invalidity" when the image data is determined to be invalid.

The valid image data mentioned above is image data with little noise, and is frame-based image data where the number of lines in one frame is equal to or larger than a predetermined threshold. On the other hand, the invalid image data is image data with many noises, and is frame-based image data where the number of lines in one frame is less than the predetermined threshold.

The data generator 41 serves to generate data for output. The data for output is an image signal consists of the binarization signal S1 and the result of determination that is included in the determination result signal S2 and added into a blanking portion of the binarization signal S1. Specifically, the data generator 41 receives the binarization signal S1 via the AND gate 38, detects the blanking portion of the binarization signal S1, receives the determination result signal S2 from the image determining unit 40, and adds the determination result signal S2 to the blanking portion of the binarization signal S1. The blanking portion is a portion corresponding to a blanking period and placed immediately after the frame-based image data included in the binarization signal S1. The data generator 41 adds the determination result signal S2 corresponding to the result of determination on the image data to the blanking portion placed immediately after the image data. The output timing of the binarization signal S1 is controlled by the AND gate 38 as described earlier. Hence, the data generator 41 can readily detect the blanking portion of the binarization signal S1 for each frame, and add the determination result signal S2 to the blanking portion without losing the image data. Thereafter, the data generator 41 supplies an additional binarization signal S3 (in other words, the data for output mentioned above), in which the determination result signal S2 is added to the blanking portion of the binarization signal S1, to the AND gate 43.

The connection detector 42 serves to detect the connection between the receiving apparatus 3 and the monitor apparatus 7. Specifically, the connection detector 42 detects that the receiving apparatus 3 is connected to the monitor apparatus 7 by detecting an electrical conduction caused by the connection between the receiving apparatus 3 and the monitor apparatus 7 by the cable 8. The connection detector 42 supplies a high-level signal to the AND gate 43 on detecting the connection between the receiving apparatus 3 and the monitor apparatus 7. When the AND gate 43 receives the additional binarization signal S3 from the data generator 41 and the high-level signal from the connection detector 42, the AND gate 43 supplies the additional binarization signal to a differential transmission I/F 44. In other words, the additional binarization signal is supplied to the differential transmission I/F 44 when the receiving signal 3 is connected to the monitor apparatus 7.

The differential transmission I/F 44 serves to transmit the image data obtained by the receiving apparatus 3 and the result of determination on the image data to the monitor apparatus 7 in a serial form. Specifically, the differential transmission I/F 44 is implemented with a serial interface that supplies serial signals to the outside according to a differential method such as LVDS (Low Voltage Differential Signaling), and the differential transmission I/F 44 is connected to the monitor apparatus 7 via the cable 8. The cable 8 is a cable adoptable for differential transmission and has a pair of differential transmission lines D+ and D-. The differential transmission I/F 44 supplies the additional binarization signal S3 supplied via the AND gate 43 to the cable 8. The additional binarization signal S3 thus supplied from the differential transmission I/F 44 is transmitted in a serial form to the monitor apparatus 7 via the cable 8 according to the differential method. The additional binarization signal S3 (data for output) is, as described above, a serial signal in which the determination result signal S2 is added into the blanking portion of the binarization signal S1. In other words, the additional binarization signal S3 is an image signal in which the result of determination on the image data is added to the blanking portion which is located immediately after the frame-based image data.

The display unit 45 works to display various types of information under the control of the control unit 47, and displays information concerning the subject 1, for example. The information concerning the subject 1 is, for example, patient name of the subject 1, patient ID which identifies the subject 1, age, sex, and date of examination (date the subject 1 swallows the capsule endoscope 2). The display unit 45 has a function of receiving input information like a touch panel, and supplies information to the control unit 47 to give an instruction to the control unit 47.

To the storage unit 46, the portable recording medium 5 can be detachably attached. The storage unit 46 sequentially stores the information such as image data generated by the image processing unit 39 into the portable recording medium 5 according to a recording instruction from the control unit 47. The storage unit 46 may be provided with a memory IC such as a RAM or a flash memory and be configured so as to store information such as the image data in the storage unit 46 itself.

The control unit 47 is implemented with a CPU (Central Processing Unit) that executes a processing program, a ROM in which the processing program and the like is previously stored, and a RAM in which various operation parameters for the processing and various input information and the like supplied to the control unit 47 are stored. The control unit 47 controls the driving of each element in the receiving apparatus 3. For example, the control unit 47 performs a data input/output control of the display unit 45, and also controls a display operation of the display unit 45, and a data storing operation, data reading operation, and the like of the storage unit 46, for example. Further, the control unit 47 controls so as to delete the image data which is determined to be invalid by the image determining unit 40 without storing the same in the storage unit 46, and to store the image data which is determined to be valid by the image determining unit 40 into the storage unit 46. The control unit 47 receives the image data generated by the image processing unit 39, and also receives the result of determination on the validity/invalidity of the image data from the image determining unit 40. When the result of determination indicates validity, the control unit 47 stores the image data as the valid image data in the storage unit 46, whereas when the result of determination indicates invalidity, the control unit 47 deletes the image data as invalid image data. As can be seen from the above, the result of determination supplied to the control unit 47 by the image determining unit 40 is, as described above, determination on validity/invalidity of the image data, as well as determination on whether to store the image data supplied to the control unit 47 by the image processing unit 39 into the storage unit 46 or not.

A structure of the monitor apparatus 7 will be described. As shown in FIG. 2, the monitor apparatus 7 includes a differential transmission I/F 71 which receives the image data and the result of determination on the image data from the receiving apparatus 3 via the cable 8, a connection detector 72 which detects the connection between the receiving apparatus 3 and the monitor apparatus 7, a switching circuit 73 which outputs one of an image signal received from the capsule endoscope 2 not via the receiving apparatus 3 and an image signal received from the receiving apparatus 3 via the differential transmission I/F 71, an image processing unit 74 which generates image data based on an image signal supplied from the switching circuit 73, and a determination result detector 75 which detects a result of determination added to the image signal for the image data. The monitor apparatus 7 further includes a display unit 76 which displays image data generated by the image processing unit 74 and information indicating whether the image data is stored in the receiving apparatus 3 or not, a control unit 77 which controls the driving of each element in the monitor apparatus 7, and a power supply unit 78 which supplies driving power to each element in the monitor apparatus 7.

The monitor apparatus 7 has a function of receiving image signals from the capsule endoscope 2 not via the receiving apparatus 3. Specifically, the monitor apparatus 7 includes a receiving antenna 80 that receives the radio signals from the capsule endoscope 2, a demodulator 81 that demodulates the radio signals received via the receiving antenna 80 into the image signals, a synchronization detector 82 that detects a vertical synchronization signal included in the image signal demodulated by the demodulator 81, a binarizing unit 83 that binarizes the image signal demodulated by the demodulator 81, and an AND gate 84 that controls an output of the image signal binarized by the binarizing unit 83. The AND gate 84 supplies the image signal binarized by the binarizing unit 83 to the switching circuit 73.

The differential transmission I/F 71 serves to receive the image data transmitted in a serial form from the receiving apparatus 3 via the cable 8 and the result of determination on the image data. Specifically, the differential transmission I/F 71 is implemented with a serial interface which receives signals transmitted in a serial form according to a differential method such as LVDS. The differential transmission I/F 71 is connected to the differential transmission I/F 44 of the receiving apparatus 3 via the cable 8. The differential transmission I/F 71 receives the additional binarization signal S3 transmitted in a serial form from the differential transmission I/F 44 via the cable 8, and outputs the supplied additional binarization signal S3 to the switching circuit 73.

The connection detector 72 serves to detect the connection between the receiving apparatus 3 and the monitor apparatus 7. Specifically, the connection detector 72 detects that the receiving apparatus 3 is connected to the monitor apparatus 7 by detecting an electrical conduction caused by the connection between the receiving apparatus 3 and the monitor apparatus 7 via the cable 8. The connection detector 72 supplies a result of detection indicating that the receiving apparatus 3 and the monitor apparatus 7 are connected to the control unit 77 on detecting the connection between the receiving apparatus 3 and the monitor apparatus 7.

The switching circuit 73 performs a switching operation so as to electrically connect the differential transmission I/F 71 to the image processing unit 74 and the determination result detector 75 when the connection detector 72 detects the connection between the receiving apparatus 3 and the monitor apparatus 7, and to electrically connect the AND gate 84 and the image processing unit 74 when the connection between the receiving apparatus 3 and the monitor apparatus 7 is not detected. In other words, the additional binarization signal S3 received from the receiving apparatus 3 via the differential transmission I/F 71 is supplied to the image processing unit 74 and the determination result detector 75 via the switching circuit 73 when the receiving apparatus 3 and the monitor apparatus 7 are connected with each other via the cable 8. On the other hand, the image signal binarized by the binarizing unit 83 (in other words, the image signal received from the capsule endoscope 2 not via the receiving apparatus 3) is supplied to the image processing unit 74 and the determination result detector 75 via the switching circuit 73 when the receiving apparatus 3 and the monitor apparatus 7 are not connected with each other.

The image processing unit 74 serves to generate image data to be displayed on a screen of the display unit 76. Specifically, the image processing unit 74 receives the additional binarization signal S3 from the differential transmission I/F 71 via the switching circuit 73, and generates the frame-based image data by performing predetermine image processing and the like on the received additional binarization signal S3. The image data here, is the image data acquired by the receiving apparatus 3 as described above. The image processing unit 74 supplies thus generated image data to the control unit 77.

The determination result detector 75 serves to detect the result of determination of the image data acquired via the receiving apparatus 3. Specifically, the determination result detector 75 receives the additional binarization signal S3 from the differential transmission I/F 71 via the switching circuit 73 and detects the result of determination included in the received additional binarization signal S3 for the image data. The result of determination included in the additional binarization signal S3 for the image data indicates the validity/invalidity of the image data (in other words, whether the image data is stored in the receiving apparatus 3 or not), and is indicated by a high-level signal or a low-level signal added in the blanking portion of the image signal as described above. The determination result detector 75 supplies the detected result of determination on the image data to the control unit 77. The result of determination supplied to the control unit 77 by the determination result detector 75 may indicate validity/invalidity of the image data, or alternatively, may indicate whether the image data is stored in the receiving apparatus 3 or not.

The display unit 76 serves to display the image data acquired by the receiving apparatus 3 on the screen. Specifically, the display unit 76 is implemented with a liquid crystal display or an organic EL display, for example, and displays various types of information for which the control unit 77 gives a displaying instruction. The display unit 76 displays, for example, the image data generated by the image processing unit 74 and information corresponding to the result of determination detected by the determination result detector 75. The information corresponding to the result of determination is, for example, information indicating whether the image data displayed on the screen is stored in the receiving apparatus 3 or not. The display unit 76 may have a function of receiving information input as in a touch panel or the like, and may serve to supply information to the control unit 77 to give instruction to the control unit 77.

The control unit 77 is implemented with a CPU that executes a processing program, a ROM in which the processing program and the like is previously stored, and a RAM in which operation parameters for each processing and various input information and the like supplied to the control unit 47 are stored. The control unit 77 controls the driving of each element in the monitor apparatus 7. For example, the control unit 77 controls the data input/output of the display unit 76 and a display operation of the display unit 76. Here, the control unit 77 displays the image data generated by the image processing unit 74 at the display unit 76 and, if the connection detector 72 detects the connection between the receiving apparatus 3 and the monitor apparatus 7, displays the information corresponding to the result of determination as detected by the determination result detector 75 corresponding to the image data at the display unit 76.

Further, the control unit 77 controls the switching operation of the switching circuit 73 based on the result of detection of the connection detector 72. Specifically, the control unit 77 controls the switching operation of the switching circuit 73 so that the differential transmission I/F 71 is electrically connected to the image processing unit 74 and the determination result detector 75 when the control unit 77 receives the result of detection indicating that the connection between the receiving apparatus 3 and the monitor apparatus 7 is detected from the connection detector 72. At this time, the control unit 77 may stop the driving of the demodulator 81. Whereby, the control unit 77 can reduce the driving power consumed for useless operation of the demodulator 81 to demodulate the radio signals from the capsule endoscope 2 while the receiving apparatus 3 and the monitor apparatus 7 are connected by the cable 8.

On the other hand, the control unit 77 controls the switching operation of the switching circuit 73 so that the AND gate 84 is electrically connected to the image processing unit 74 and the determination result detector 75 when the above result of detection is not received from the connection detector 72. Then, the monitor apparatus 7 works to receive the radio signals from the capsule endoscope 2 via the receiving antenna 80 not via the receiving apparatus 3, and to display the image data based on the received radio signals on the display unit 76.

The receiving antenna 80 serves to receive the radio signals from the capsule endoscope 2 solely by the monitor apparatus 7 independent of the receiving apparatus 3, while the receiving apparatus 3 and the monitor apparatus 7 are not connected via the cable 8. Specifically, the receiving antenna 80 receives the radio signals including the image data acquired through image capturing by the capsule endoscope 2 and supplies the received radio signals to the demodulator 81.

The demodulator 81 performs processing such as demodulation on the radio signals sent from the capsule endoscope 2 and received via the receiving antenna 80, and demodulates the radio signals into the image signals, which are base band signals, similarly to the demodulator 35 of the receiving apparatus 3 described above. The demodulator 81 supplies the resulting image signal to the synchronization detector 82 and the binarizing unit 83.

The synchronization detector 82 detects the vertical synchronization signal included in the image signal supplied from the demodulator 81 for each frame. Every time the vertical synchronization signal is detected, the synchronization detector 82 supplies a high-level signal to the AND gate 84. The binarizing unit 83 binarizes the image signal supplied from the demodulator 81 and supplies the binarization signal, which is the binarized image signal, to the AND gate 84, similarly to the binarizing unit 37 of the receiving apparatus 3 described earlier.

The AND gate 84 supplies the binarizing signal supplied from the binarizing unit 83 to the switching circuit 73 when the synchronization detector 82 supplies the high-level signal as mentioned above. In other words, the AND gate 84 supplies the binarization signal from the binarizing unit 83 to the switching circuit 73 at the timing of detection of the vertical synchronization signal of the image signal by the synchronization detector 82.

When the receiving apparatus 3 and the monitor apparatus 7 are not connected with each other, the switching circuit 73 electrically connects the AND gate 84 with the image processing unit 74 under the control of the control unit 77 as described above. The image processing unit 74 receives the binarization signal from the binarizing unit 83 via the switching circuit 73, and generates the frame-based image data by performing predetermined image processing on the binarization signal. The image data here, is image data acquired through image capturing by the capsule endoscope 2, and is image data obtained by the monitor apparatus 7 alone and not via the receiving apparatus 3. The image processing unit 74 supplies thus generated image data to the control unit 77. The control unit 77 displays the image data generated by the image processing unit 74 at the display unit 76.

On the other hand, the binarization signal supplied from the binarizing unit 83 is supplied to the determination result detector 75 via the switching circuit 73. However, the supplied binarization signal does not include the result of determination on the image data as the additional binarization signal S3 mentioned above. Hence, the determination result detector 75 has difficulties in detecting a correct determination result on the image data included in the binarization signal supplied from the binarizing unit 83. Hence, when the control unit 77 does not receive the result of detection indicating that the connection of the receiving apparatus 3 and the monitor apparatus 7 is detected from the connection detector 72, the control unit 77 may stop the driving of the determination result detector 75, or alternatively, may delete the result of detection supplied from the determination result detector 75 (in other words, cancel the result of detection). Alternatively, when the control unit 77 does not receive the result of detection indicating that the connection between the receiving apparatus 3 and the monitor apparatus 7 is detected from the connection detector 72, the control unit 77 may display the image data generated by the image processing unit 74 at the display unit 76, and at the same time may display information indicating that the displayed image data is acquired solely by the monitor apparatus 7 not via the receiving apparatus 3 at the display unit 76.

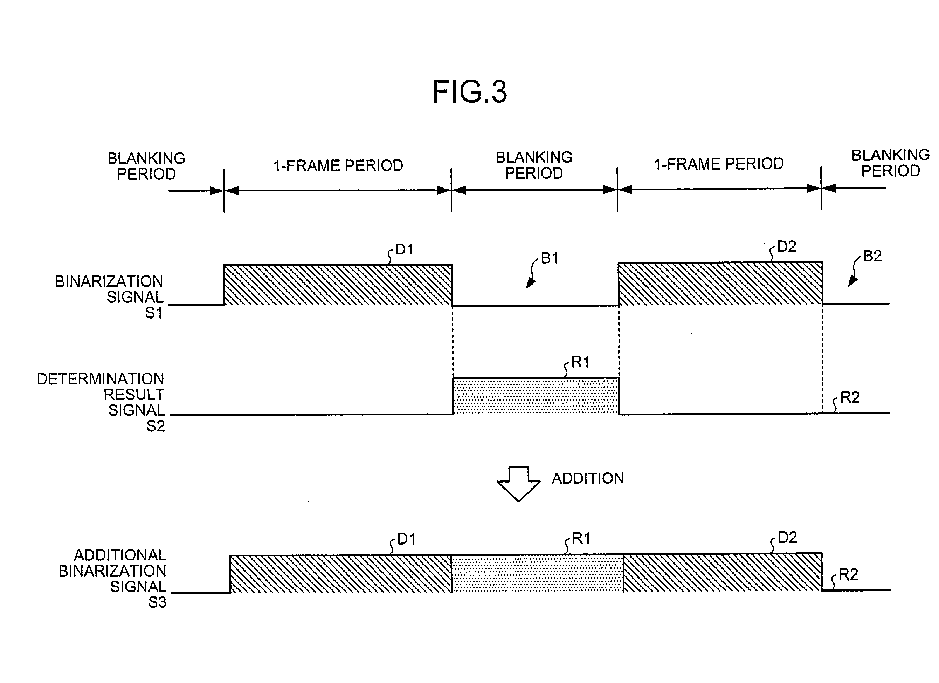

The operation of the data generator 41 which adds the result of determination of the image data to the blanking portion of the binarization signal S1 to generate the data for output mentioned above will be described. FIG. 3 is a schematic diagram describing the operation of generating the additional binarization signal S3 which is the data for output in which the result of determination of the image data is added to the binarization signal S1. As shown in FIG. 3, the binarization signal S1 is a serial signal in which one-frame period including the image data or the like and a blanking period not including the image data or the like are alternately disposed. The binarization signal S1 includes frame data D1 and D2 in the respective one-frame periods, and blanking portions B1 and B2 in the respective blanking periods subsequent to the frame data D1 and D2, respectively. The frame data is data including image data of one frame, the vertical synchronization signal, the horizontal synchronization signal, and the parameters such as a white balance coefficient.

An exemplary operation performed when the image determining unit 40 determines that the image data included in the frame data D1 is valid and the image data included in the frame data D2 is invalid will be described. The data generator 41 receives the binarization signal S1 including the frame data D1 and D2 via the AND gate 38, and receives the determination result signal S2 from the image determining unit 40. In the determination result signal S2, the determination result data indicating that the image data of the frame data D1 is valid (i.e., high-level signal) and the determination result data indicating that the image data of the frame data D2 is invalid (i.e., low-level signal) are arranged at predetermined intervals.

Thereafter, the data generator 41 detects the blanking portions B1 and B2 of the received binarization signal S1, and adds determination result data R1 and R2 of the determination result signal S2 to the blanking portions B1 and B2, respectively, as shown in FIG. 3. Since the output timing of the binarization signal S1 is controlled by the AND gate 38, the data generator 41 can readily detect the blanking portions B1 and B2, and add the determination result data R1 and R2 to the blanking portions B1 and B2 without losing the image data of the frame data D1 and D2 (for example, without overlapping the frame data D1 and D2 with the determination result data R1).

Thus, the data generator 41 generates the additional binarization signal S3 by adding the determination result signal S2 to the binarization signal S1. In the additional binarization signal S3, the determination result data R1 is arranged after the frame data D1 and the determination result data R2 is arranged after the frame data D2, as shown in FIG. 3. In other words, the additional binarization signal S3 includes the frame-based image data in each of the frame data D1 and D2, and the determination result indicating that the image data of the frame data D1 is valid (image data to be stored in the receiving apparatus 3), and the determination result indicating that the image data of the frame data D2 is invalid (image data not to be stored in the receiving apparatus 3). The data generator 41 supplies thus generated additional binarization signal S3 to the AND gate 43.

Even when the data generator 41 receives a binarization signal including one or more frame data, the data generator 41 generates the additional binarization signal S3 by adding the determination result signal S2, substantially in the same manner as on receiving the binarization signal S1 including the frame data D1 and D2 mentioned above.

The receiving apparatus 3 having the above described structure can transmit in a serial form the image signal to which the determination result on the image data is added by the data generator 41 to the monitor apparatus 7. Therefore, the number of cables (in other words, the number of signal transmission lines) for transmitting the image signals or the like to the monitor apparatus 7 can be reduced in comparison with the number thereof in the conventional receiving apparatus which outputs respective color signals and the synchronization signal (horizontal synchronization signal and vertical synchronization signal) of the stored image data to the monitor apparatus. Thus, the EMI (Electro Magnetic Interference) at the transmission of the image signals or the like from the receiving apparatus 3 to the monitor apparatus 7 can be reduced, i.e., noise resistance can be improved.

Further, since the receiving apparatus 3 adds the determination result of the image data to the image signal obtained as a result of demodulation of the radio signals transmitted from the capsule endoscope 2, and transmits the resulting signal in a serial form to the monitor apparatus 7, a signal generating circuit for generating color signals (R, G, B) and synchronization signals of the stored image data can be eliminated. Specifically, three operational amplifiers for providing a gain for the respective color signals and a crystal oscillator for generating the synchronization signals, for example, can be eliminated. Therefore, the receiving apparatus 3 can transmit the image data obtained from the capsule endoscope 2 and the determination result obtained by the image determining unit 40 to the monitor apparatus 7 in a serial form, and at the same time, the circuit scale of the receiving apparatus 3 can be downsized in comparison with the circuit scale of the conventional receiving apparatus which incorporates a signal generating circuit like the one described above.

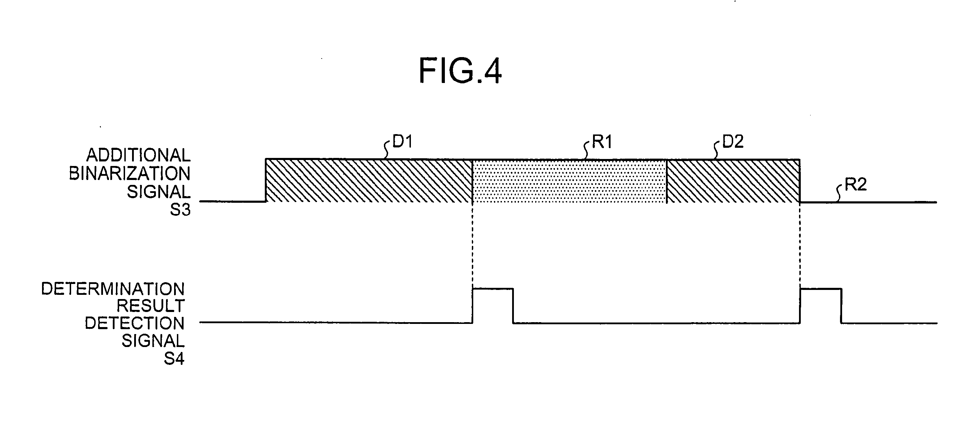

The operation of the determination result detector 75 which detects the determination result included in the additional binarization signal S3, which is transmitted in a serial form from the receiving apparatus 3, of the image data will be described. FIG. 4 is a schematic diagram describing the detecting operation of the determination result of the image data included in the additional binarization signal S3. As shown in FIG. 4, the determination result detector 75 employs a determination result detection signal S4 to detect a high-level signal or a low-level signal at intervals corresponding to the blanking period of the binarization signal S1, thereby detecting the determination result of the image data included in the additional binarization signal S3.

For example, when the determination result detector 75 receives the additional binarization signal S3 including the frame data D1 and D2 mentioned above, the determination result detector 75 sequentially detects the determination result data R1 and the determination result data R2 employing the determination result detection signals S4 as shown in FIG. 4. The determination result detector 75 detects the determination result data R1 as a high-level signal, and supplies the determination result indicating that the image data in the frame data D1 which is arranged immediately before the determination result data R1 is valid image data (i.e., image data stored in the receiving apparatus 3) to the control unit 77. Further, the determination result detector 75 detects the determination result data R2 as a low-level signal, and supplies the determination result indicating that the image data in the frame data D2 arranged immediately before the determination result data R2 is invalid image data (i.e., image data not stored in the receiving apparatus 3) to the control unit 77.

Duty ratio of the binarization signal S1 mentioned above may fluctuate (for example, there might be a narrow pulse width) depending on the degradation in the sensitivity of the receiving apparatus 3 at the reception of the radio signals from the capsule endoscope 2. As a result, if both the receiving apparatus 3 and the monitor apparatus 7 perform the determining process to determine the validity/invalidity of the image data included in the binarization signal S1, the determination results obtained by the receiving apparatus 3 and the monitor apparatus 7 for the image data may not coincide with each other, even when the same binarization signal S1 is processed. Such unconformity is attributable to the tolerance of the crystal oscillator incorporated in each of the receiving apparatus 3 and the monitor apparatus 7. Because of the tolerance of the crystal oscillators, when the receiving apparatus 3 and the monitor apparatus 7 detect the horizontal synchronization signals of the same binarization signal S1, the detected numbers of the horizontal synchronization signals sometimes differ.

The determination result detector 75, on the other hand, can detect the determination result of the image data based on the additional binarization signal S3 without the need of determination on the validity/invalidity of the image data based on the number of detected horizontal synchronization signals, dissimilar to the image determining unit 40. The determination result data included in the additional binarization signal S3 corresponds to the determination result of the image data which is already provided by the image determining unit 40 described above. Therefore, the determination result detector 75 can detect the same determination result made by the image determining unit 40 for the image data based on the additional binarization signal S3. The control unit 77 can obtain the same determination result from the determination result detector 75 for the image data as the determination result made by the image determining unit 40, for each image data acquired by the receiving apparatus 3.

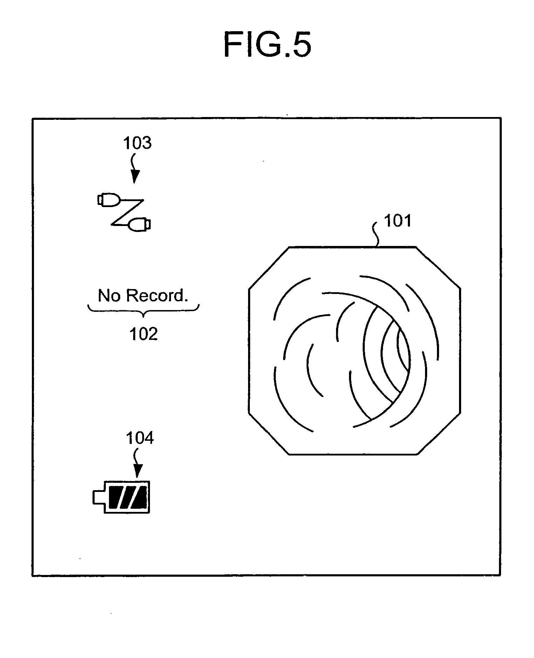

The display on the screen by the display unit 76 will be specifically described. FIG. 5 is a schematic diagram of a specific example of a content of display by the display unit 76. The display unit 76 displays various types of information on the screen under the control of the control unit 77. The display unit 76 displays, as shown in FIG. 5, for example, image data 101 obtained through image capturing by the capsule endoscope 2, storage information 102 indicating whether the image data 101 is stored in the receiving apparatus 3 or not, connection state information 103 indicating that the receiving apparatus 3 and the monitor apparatus 7 are in a connected state, and remaining-battery-amount information 104 indicating the remaining amount of the driving power of the monitor apparatus 7.

The display unit 76 sequentially displays the image data obtained via image capturing by the capsule endoscope 2 as shown by the image data 101 of FIG. 5, for example. When the image data 101 displayed on the screen is one that acquired by the receiving apparatus 3 (in other words, the image transmitted from the receiving apparatus 3 to the monitor apparatus 7), the display unit 76 displays the storage information 102 together with the image data 101. The storage information 102 is information indicating whether the pertinent image data 101 is stored in the receiving apparatus 3 or not, and corresponds to the determination result given by the image determining unit 40 on validity/invalidity of the image data 101. When the image data 101 is not stored in the receiving apparatus 3 (in other words, when the image data is invalid), the storage information 102 is displayed as information indicating that the image data is not stored in the receiving apparatus 3 (for example, "No Record.") as shown in FIG. 5. On the other hand, when the image data 101 is stored in the receiving apparatus 3 (in other words, when the image data is valid), the storage information 102 is displayed as information indicating that the image data is stored in the receiving apparatus 3 (for example, "Record.").

Further, the display unit 76 displays the connection state information 103 indicating that the receiving apparatus 3 is connected to the monitor apparatus 7 via the cable 8, if the receiving apparatus 8 and the monitor apparatus 7 are connected via the cable 8. Further, the display unit 76 displays the remaining-battery-amount information 104, and changes the displayed remaining-battery-amount information 104 according to the actual remaining battery amount of the driving power of the monitor apparatus 7.

Further, the storage information 102 may be displayed as information indicating that the image data 101 displayed on the same screen is not stored in the receiving apparatus 3 ("No Record." in FIG. 5) when that is the case, whereas the storage information 102 may not be displayed when the image data 101 is stored in the receiving apparatus 3. In other words, the storage information 102 may be information indicating that the image data 101 is not stored in the receiving apparatus 3. On the contrary, the storage information 102 may be information indicating that the image data 101 is stored in the receiving apparatus 3, and displayed only when the image data 101 displayed on the same screen is stored in the receiving apparatus 3.

The doctor, the nurse, or the like can monitor the image data (image data of the subject 1, for example) acquired by the receiving apparatus 3 from the capsule endoscope 2 on real time, and correctly confirm whether the image data displayed on the screen is stored in the storage unit 46 of the receiving apparatus 3 or not by checking the contents displayed by the display unit 76. Further, the doctor, the nurse, or the like can confirm the connection state of the receiving apparatus 3 and the monitor apparatus 7, and further can readily confirm the remaining amount of the driving power of the monitor apparatus 7.

The display unit 76 can display the image data acquired solely by the monitor apparatus 7, i.e., the image data received from the capsule endoscope 2 via the receiving antenna 80 on the screen, instead of the image data 101 acquired via the receiving apparatus 3. Then, the doctor, the nurse, or the like can monitor the image data inside the subject 1 as acquired through image capturing by the capsule endoscope 2 on real time without the relay of the receiving apparatus 3. Still further, the doctor, the nurse, or the like can confirm whether the capsule endoscope 2 operates normally or not by monitoring the image data of the capsule endoscope 2 before the capsule endoscope 2 is swallowed by the subject 1.

In the embodiment of the present invention, the validity/invalidity of the image data is determined based on the number of the detected horizontal synchronization signals included in the image signal. The present invention, however, is not limited thereto. Alternatively, the validity/invalidity of the image data may be determined based on whether the vertical synchronization signal in the image signal is detected or not, or whether the strength of the received radio signal transmitted from the capsule endoscope 2 is not less than a predetermined threshold or not. Further, the validity/invalidity of the image data may be determined based on whether the detected white balance coefficient included in the image signal takes a value within a predetermined acceptable range or not. Still alternatively, identification information such as an ID identifying the capsule endoscope 2 may be superposed onto the image signal in advance, and validity/invalidity of the image data may be determined based on whether the identification information is detected from the image signal or not.

In the embodiment of the present invention, the image determining unit 40 outputs a high-level signal as the determination result indicating the valid image data, and outputs a low-level signal as the determination result indicating the invalid image data. The present invention, however, is not limited thereto. The image determining unit 40 may output a low-level signal as the determination result indicating the valid image data, and outputs a high-level signal as the determination result indicating the invalid image data. Then, the determination result detector 75 may be configured so as to detect the determination result indicating the valid image data by detecting the low-level signal superposed in the blanking portion, and to detect the determination result indicating the invalid image data by detecting the high-level signal superposed in the blanking portion.

Further, the data generator 41 adds the information on necessity of recording, i.e., the determination result on whether the image data is a target of recording into the storage unit 46 of the receiving apparatus 3 or not (in other words, whether the image data is valid image data or not), to the image signal corresponding to the pertinent image data. The present invention, however, is not limited thereto. For example, the information on necessity of recording (i.e., information corresponding to the determination result on validity/invalidity of the image data) may be superposed onto the image data, and the data for output may be generated so as to include the image data and the information on necessity of recording.

In the embodiment of the present invention, the differential transmission I/F is employed as a communication I/F for connecting the receiving apparatus 3 and the monitor apparatus 7. The present invention, however, is not limited thereto, and the USB or a serial I/F such as RS232C can be employed as the communication I/F. Accordingly, the cable 8 may be any serial transmission cable to which the above serial I/F can be connected. Further, the serial I/F is desirably a differential transmission I/F such as LVDS mentioned above, because the use of the differential transmission I/F enhances the noise resistance of the receiving apparatus 3 and the monitor apparatus 7 at the serial transmission, and contributes to the reduction in power consumption.

As described above, in the embodiment of the present invention, the validity/invalidity of the image data is determined based on the image signal including the image data acquired through image capturing by the capsule endoscope, the data for output is generated as an image signal including the above image signal and the determination result of the image data added to the blanking portion of the image signal, and the data for output is output by serial transmission. Hence, the image data for which the determining process is performed to determine whether the image data is stored in the storage unit or not and the determination result can be supplied to the outside by serial transmission. Therefore, it is not necessary to incorporate the circuit for generating the color signals (R, G, B) and the synchronization signal of the valid image data stored in the storage unit, and accordingly the number of cables for transmitting the image data or the like to the outside can be reduced. Thus, the receiving apparatus which can output the image data transmitted from the capsule endoscope and the information on whether the image data is stored or not to the outside can be downsized and made to consume less power.

Further, the monitor apparatus according to the present invention is configured so as to have a serial interface for receiving the image signal to which the determination result is added by the receiving apparatus according to the present invention, to detect the determination result of the image data included in the image signal received via the serial interface, and display the image data and the information corresponding to the determination result (information on whether the image data is stored or not) on the same screen. Therefore, dissimilar to the receiving apparatus, the monitor apparatus can acquire the same determination result as the determination result on the image data obtained by the receiving apparatus without determining the validity/invalidity of the image data, allows for the monitoring of the image data acquired by the receiving apparatus on real time, and correctly confirm whether the image data displayed on the screen is stored in the receiving apparatus or not, and such monitor apparatus can be downsized and made to consume less power.

When the above receiving apparatus and the above monitor apparatus are connected, the intra-subject information acquiring system can be realized according to which the image data acquired through image capturing by the capsule endoscope inside the subject can be acquired, the downsizing of the apparatus scale and the reduction of power consumption can be facilitated, and the monitoring of the image data acquired by the receiving apparatus and the confirmation of whether the image data is stored in the receiving apparatus or not can be correctly performed.

### INDUSTRIAL APPLICABILITY

The receiving apparatus, the monitor apparatus, and the intra-subject information acquiring system according to the present invention is useful for the reception and the monitoring of the image data acquired through image capturing of the capsule endoscope inside the subject, and particularly appropriate for further downsizing of the apparatus scale and for monitoring whether the image data transmitted from the capsule endoscope is stored in the recording medium as valid image data or not.

## Claims

1. A receiving apparatus comprising:
a receiving unit that receives image data;
a determining unit that is connected to a subsequent stage of the receiving unit and determines whether the image data acquired is a target of record or not;
a data generator that generates data for output to which information on necessity of recording is included, the information being a result of determination by the determining unit; and
an interface that outputs the data for output.

2. The receiving apparatus according to claim 1, wherein
the data generator generates the data for output by adding the information on necessity of recording to the image data.

3. The receiving apparatus according to claim 1 or 2, wherein
the interface is a serial interface.

4. The receiving apparatus according to claim 3, wherein
the serial interface is a differential transmission interface.

5. The receiving apparatus according to claim 1, comprising:
a binarizing unit that binarizes the image data, wherein
the data generator adds the information on necessity of recording to the image data binarized by the binarizing unit.

6. The receiving apparatus according to claim 1, wherein
the determining unit detects a horizontal synchronization signal included in an image signal which includes the image data, and determines that the image data is the target of record when a number of the horizontal synchronization signals detected is equal to or larger than a predetermined threshold.

7. A monitor apparatus comprising:
an interface that receives radio signals including image data acquired through image capturing by a capsule endoscope introduced into a subject, acquires the image data based on the radio signals, determines whether the image data acquired is valid image data or not, and receives image signals which includes the image data acquired by a receiving apparatus and a result of determination on the image data, the interface being connected to the receiving apparatus which stores the valid image data therein;
a detector that detects the result of determination included in the image signal;
a display unit that displays the image data and information corresponding to the result of determination, the image data and the information being included in the image signal; and
a control unit that controls to display the image data and the information corresponding to the result of determination based on the result of determination detected by the detector, the image data and the information being included in the image signal.

8. The monitor apparatus according to claim 7, wherein the interface is a serial interface.

9. The monitor apparatus according to claim 8, wherein
the serial interface is a differential transmission interface.

10. The monitor apparatus according to any one of claims 7 to 9, wherein
the information corresponding to the result of determination is information indicating whether the image data is stored in the receiving apparatus or not.

11. An intra-subject information acquiring system including: a receiving apparatus that receives radio signals including image data acquired through image capturing by a capsule endoscope introduced into a subject, demodulates the radio signals received into image signals, and stores valid image data among the image data acquired based on the image signals; and a monitor apparatus that is connected to the receiving apparatus via an interface, and displays the image data acquired by the receiving apparatus, wherein
the receiving apparatus determines whether the image data acquired is a target of record or not, generates data for output including a result of determination and the image data, and transmits the acquired data for output to the monitor apparatus via the interface, and
the monitor apparatus receives the data for output via the interface, detects the result of determination included in the data for output received, and displays information corresponding to the result of determination detected together with the image data.

12. The intra-subject information acquiring system according to claim 11, wherein
the interface is a serial interface.

13. The intra-subject information acquiring system according to claim 12, wherein
the serial interface is a differential transmission interface.

14. The intra-subject information acquiring system according to any one of claims 11 to 13, wherein
the information corresponding to the result of determination is information indicating whether the image data is stored in the receiving apparatus or not.
